# EUROPEAN PATENT SPECIFICATION

(11) **EP 2 440 937 B1**
(45) Date of publication and mention of the grant of the patent: **29.10.2014**
(21) Application number: 10785606.4
(22) Date of filing: 11.06.2010
(51) Int. Cl.: G01N 33/92, G01N 33/574

(54) **A DIAGNOSTIC METHOD**
DIAGNOSEVERFAHREN
MÉTHODE DE DIAGNOSTIC

(30) Priority: 12.06.2009 AU 2009902730
(43) Date of publication of application: 18.04.2012
(73) Proprietor: SBC Research Pty Ltd, Sydney, New South Wales 2000 (AU)
(72) Inventor: FRENCH, Peter William, Balmain New South Wales 2041 (AU); MISTRY, Dharmica April Harridatt, Jannali New South Wales 2226 (AU); HAKLANI, Joseph, Guildford New South Wales 2161 (AU); CORINO, Gary L., Curlewis, Victoria 3222 (AU)
(74) Representative: HOFFMANN EITLE
(86) International application number: PCT/AU2010/000727
(87) International publication number: WO 2010/141998

(56) References cited:
- WO-A1-00/34774
- WO-A1-2008/000020
- WO-A1-2008/004665
- WO-A1-2008/122805
- WO-A2-2005/094327
- HYE KYUNG RYU ET AL: "Determination of cholesterol in human hair using gas chromatography-mass spectrometry", BIOMEDICAL CHROMATOGRAPHY, vol. 20, no. 10, 16 March 2006 (2006-03-16), pages 999-1003, XP055060854, ISSN: 0269-3879, DOI: 10.1002/bmc.615
- TAKEHISA FUJIWAKI ET AL: "Application of delayed extraction matrix-assisted laser desorption ionization time-of-flight mass spectrometry for analysis of sphingolipids in cultured skin fibroblasts from sphingolipidosis patients", BRAIN AND DEVELOPMENT, vol. 24, no. 3, 1 April 2002 (2002-04-01), pages 170-173, XP055060872, ISSN: 0387-7604, DOI: 10.1016/S0387-7604(02)00026-8
- LYMAN D ET AL: "Fourier Transform Infrared attenuated total reflection analysis of human hair: Comparison of hair from breast cancer patients with hair from healthy subjects", APPLIED SPECTROSCOPY, THE SOCIETY FOR APPLIED SPECTROSCOPY. BALTIMORE, US, vol. 59, no. 1, 1 January 2005 (2005-01-01), pages 26-32, XP008100668, ISSN: 0003-7028, DOI: 10.1366/0003702052940440
- MISTRY ET AL: "Identification of Breast Cancer-Associated Lipids in Scalp Hair", BREAST CANCER: BASIC AND CLINICAL RESEARCH, 1 July 2012 (2012-07-01), page 113, XP055060857, DOI: 10.4137/BCBCR.S9607
- CORINO, G. L. ET AL.: 'Diagnosis of breast cancer by X-ray diffraction of hair' INT. J. CANCER vol. 122, 2008, pages 847 - 856, XP002567867

## Description

### Field of the Invention

The present invention relates to methods to identify one or more conditions in a subject. In a particular embodiment, it relates to methods of identifying a condition which changes a lipid profile in a keratin-containing component of a subject.

### Background Art

Any discussion of the prior art throughout the specification should in no way be considered as an admission that such prior art is widely known or forms part of common general knowledge in the field.

Methods for early stage detection of disease in order to maximize treatment outcomes are clearly desirable. Cancer, and in particular breast cancer, is one example that clearly demonstrates excellent survival statistics when early-stage disease is treated using current therapies. However, for many patients diagnosis is made too late. If all cases of breast cancer could be detected prior to metastasis then there would be a significant reduction of both individual mortality and the economic burden on the community. One of the key imperatives in cancer research, including breast cancer research, therefore, is the need to develop more effective screening tools for the early detection of disease.

Biomarkers are biological molecules that are indicators of biological and pathological processes, or physiological and pharmacological responses to a drug treatment. Since biomarkers can be used to measure the progress of disease or the response to treatment, they have very significant potential roles to play in both the diagnosis and prognosis of disease. Ideally, in the case of breast cancer a biomarker signature would be able to detect cancer in asymptomatic patients and improve the accuracy of screening mammograms. A reliable biomarker signature may also signify new cancer, even in the setting of normal physical examination results, and would indicate further more intensive diagnostic workup and/or preventive treatment.

Reported differences in the small angle X-ray scattering (SAXS) patterns of hair from individuals with breast cancer compared to healthy subjects was reported in James et al 1999 (Nature 398: 33-34) and is the subject of WO 00/34774.

The SAXS patterns of hair from cancer patients contained a ring of comparatively low intensity which was superimposed on the normal α-keratin pattern obtained from healthy control subjects.

It has been reported that the results of James et al 1999 have been difficult to replicate. Trounova et al 2003 (X-ray Spectrometry 31: 314-318) noted that at least five teams were unable to find a correlation between the presence of a ring and the clinical state of patients. Further, Briki et al 1999 (Nature 400: 226) found that the ring was observed in all healthy subjects and only 80% of breast cancer patients.

WO 2008/122805 A1 discloses an analysis method consisting of the accurate determination of human fatty acid ethyl esters in hair, skin secretions and/or skin patches, toenails and fingernails by extraction and determination by gas chromatography/mass spectrometry or liquid chromatography/mass spectrometry to determine fatty acid ethyl esters (FAEE's).

Ryu HK et al. describe a method for determining cholesterol in human hair using gas chromatography-mass spectrometry (Ryu HK et al., Biomed Chromatogr. 2006 Oct;20(10):999-1003).

Fujiwaki T et al. disclose the application of delayed extraction matrix-assisted laser desorption ionization time-of-flight mass spectrometry for analysis of sphingolipids in cultured skin fibroblasts from sphingolipidosis patients (Fujiwaki T et al., Brain Dev. 2002 Apr;24(3):170-3).

Corino GL and French PW disclose the diagnosis of breast cancer by X-ray diffraction of hair (Corino GL and French PW, Int J Cancer. 2008 Feb 15;122(4):847-56).

As such, the precise nature of the ring seen on the SAXS pattern is unknown to date. Trounova et al 2003 concluded that the ring does not have a lipid nature and, instead, was due to calcification or heavy metal deposition.

It is an object of the present invention to overcome or ameliorate at least one of the disadvantages of the prior art, or to provide a useful alternative.

It is an object of the invention in its preferred form to provide an improved method to identify one or more conditions in a subject.

### Summary of the Invention

According to a first aspect, the present invention provides a method of identifying a neoplastic condition in a subject, the method including the steps of:
applying one or more extraction agents to the keratin-containing component to extract one or more lipids; and
identifying the one or more lipids,
wherein the identity of the one or more lipids is indicative of a subject having the neoplastic conditions.

According to a second aspect, the present invention provides a method of identifying a neoplastic condition in a subject, the method including the steps of:
applying one or more extraction agents to the keratin-containing component to extract one or more lipids; and
analysing the level of the one or more lipids;
wherein the level of the one or more lipids compared to a predetermined standard is indicative of a subject having the neoplastic condition.

According to a third aspect, the present invention provides a method of identifying a neoplastic condition in a subject, the method including the steps of:
applying one or more extraction agents to the keratin-containing component to extract one or more lipids;
identifying the one or more lipids; and
analysing the level of the one or more lipids;
wherein the identity and level of the one or more lipids compared to a predetermined standard is indicative of a subject having the neoplastic condition.

According to a fourth aspect, the present invention provides a method of identifying cancer in a subject, the method including the steps of:
applying a mixture of methanol, chloroform and water to the hair to extract one or more lipids;
measuring the mass and level of the one or more lipids by mass spectrometry; and
identifying the one or more lipids,
wherein the identity or level of the one or more lipids is indicative of cancer.

In one embodiment, the present invention provides a method of identifying a neoplastic condition in a subject, said method including:
obtaining a keratin-containing sample from the subject;
applying one or more extraction agents to said sample to extract one or more lipids from the keratin containing sample; and
identifying the lipid;
wherein the identity of the extracted lipid or lipids are indicative of a subject having the neoplastic condition.

In another embodiment, the present invention provides a method of identifying a neoplastic condition in a subject, said method including:
obtaining a keratin-containing sample from the subject;
applying one or more extraction agents to said sample to extract one or more lipids from the keratin containing sample; and
analysing the level of said one or more lipids in said sample;
comparing the level of said one or more lipids to a control;
wherein the level of said one or more lipids in said sample being outside of the control is indicative of a subject having the neoplastic condition.

The neoplastic condition may comprise a wide range of cancers and is not limited to a specific cancer, with the proviso that said cancer alters the lipid profile of the keratin. Examples of cancers include cancer of the breast, colon, lung, cervix, pancreas, stomach, vagina, oesophagus, kidney, ovary, duodenum, small intestine, rectum, salivary gland, or cecum.

In one embodiment the condition is a condition which results in an increase in the level of the one or more lipids compared to a predetermined standard. For example, the one or more lipids may be selected from lipids having mass 503.3176, 512.4602, 524.4594, 525.4528, 526.4751, 537.4822, 538.5113, 540.5266, 541.5297, 552.4901, 556.5215, 557.5250, 568.5560, 580.5202, 582.4656, 596.5884, 619.3451, 624.6197, 634.7441, 652.6506, 682.5185, 786.4917, 786.6695 and 850.5314.

In one embodiment the condition is a condition which results in a decrease in the level of the one or more lipids compared to a predetermined standard. For example, the one or more lipids may be selected from lipids having mass 508.4652, 510.3930, 510.4808, 511.4835, 524.4957, 534.4798, 536.4961, 549.4811, 550.4853, 554.4182, 555.5365, 574.5314, 581.5520, 582.4502, 583.5682, 598.4452, 616.5435, 618.5588, 619.5619, 626.4753, 643.4452, 646.5901, 670.5014, 707.6126, 715.5302, 723.5633 and 758.5524.

The condition may result in an alteration of the lipid profile in the keratin-containing component by a number of pathways. The condition may cause an alteration in the composition or in the normal metabolic pathways of one or more lipid species.

The one or more lipids which are extracted from the keratin containing sample and which are indicative of the condition, may be identified by a number of means including Thin Layer Chromatography (TLC). In a further embodiment, Mass Spectrometry (MS) is used to identify the extracted lipid. For specific lipids, probes may be constructed to bind to and identify the extracted lipid or lipids. Such probes include monoclonal antibodies specific antigens on a particular lipid(s).

The alteration in the lipid profile of the keratin-containing component may result from an alteration of any one or more of a number of lipids in the keratin. Examples include, but are not limited to: phospholipids including phosphatidylethanolamine, phosphatidylcholine, lysophosphatidylcholine, phosphatidylserine, phosphocholine; sphingolipids including sphingomyelin, sphingosine; palmitic acid, palmitoleic acid, arachidonic acid; linoleic acid and choline.

In one embodiment, the condition may alter normal phospholipid metabolism or composition in body tissues and/or fluids. In this embodiment, various metabolites in the phospholipid metabolism pathway may be altered. As an example, the metabolic pathway of choline may be altered to ultimately cause an altered composition or biosynthesis of choline phospholipids in a keratin-containing component.

In a further embodiment, the condition may cause a change in the profile of a fatty acid containing compound such as sebum in a keratin-containing component of the subject.

By "change in profile" it is to be understood that this term includes a number of embodiments. For example, the keratin-containing component may comprise one or more lipids not normally found in keratin-containing components of healthy subjects. Alternatively, the change in profile may comprise one or more lipids normally found in keratin of healthy subjects but wherein the level of lipid in the keratin is altered (i.e., it may be increased or decreased).

The keratin-containing component of the present invention may include, but is not limited to any type of body hair, nail, skin and cuticle. The keratin-containing sample may be taken from any of these components.

The extraction agent may be any agent which extracts lipids. For example, the extraction agent may be any organic solvent which solubilises lipids, or any combination of solvents. Examples include non-polar and polar solvents. Non-polar solvents include chloroform, hexane, toluene, benzene, diethyl ether and ethyl acetate. Polar solvents include methanol, ethanol, acetone, n-propanol, isopropanol, butanol, acetic acid, formic acid, dimethyl sulfoxide, dimethylformamide, acetonitrile, tetrahydrofuran and 1,4-dioxane. In one embodiment the extraction agent includes chloroform and methanol.

In one embodiment, the use of an extraction agent on the keratin-containing material from a subject extracts a lipid or lipids, the presence or the quantity of which is indicative of breast cancer. The means to identify the presence and/or quantity of lipids could be by any of a number of methods including but not limited to Thin Layer Chromatography, Mass Spectrometry, Gas Chromatography and High Performance Liquid Chromatography.

In an embodiment wherein the condition comprises breast cancer, the extracted lipid may be a phospholipid such as phosphatidylcholine, lysophosphatidylcholine, phosphocholine or phosphatidylethanolamine.

If the lipid profile of a keratin-containing sample is altered due to a subject suffering from a condition which alters this lipid profile, the use of a specific agent that extracts the lipid may provide a means to detect the condition earlier than by conventional means such as imaging by mammography, ultrasound or MRI.

In the context of the present invention, the term "lipid profile" refers to the characteristics of the one or more lipids extracted from the keratin-containing sample. For example, such characteristics may be the identity, mass, level, or retention time of the one or more lipids.

Lipids may be identified by any means. For example, lipids may be identified by comparing the masses of the lipids obtained by mass spectrometry to reference lipids.

In the context of the present invention, the term "predetermined standard" means the lipid level found in a corresponding keratin-containing component of a subject not having a condition which changes the lipid profile in the keratin-containing component.

The present invention provides an understanding of the underlying molecular cause of the ring seen on the SAXS pattern mentioned in James et al and provides an improved method for identifying a condition which changes a lipid profile in a keratin-containing component of a subject, such as breast cancer. This understanding and the findings set out in this invention also allow identification of conditions other than breast cancer.

### Brief Description of the Drawings

Figure 1 shows A) a normal X-ray diffraction pattern of hair and B) an abnormal X-ray diffraction pattern of hair;
Figure 2 shows A) an X-ray diffraction pattern of a hair with no ring and B) the X-ray diffraction pattern following soaking of the hair of A) in olive oil;
Figure 3 shows the effect of solvent on the X-ray diffraction pattern of a hair exhibiting a ring;
Figure 4 shows enhancement of a ring observed from the results of X-ray diffraction by using lead nitrate.
Figure 5 shows the total phosphatidylcholine/lysophosphatidylcholine extracted from hair samples from breast cancer patients and controls.
Figure 6 shows the principal component analysis scores (A) and loadings (B) for lipids extracted from hair samples from breast cancer patients and controls.
Figure 7 shows the partial least squares scores (A) and loadings (B) for lipids extracted from hair samples from breast cancer patients and controls.
Figure 8 shows the masses and retention times of lipids elevated > 2 fold in breast cancer patients (A) and elevated > 2 fold in controls (B).

### Description of an Exemplary Embodiment of the Invention

Preferred embodiments of the invention will now be described, by way of example only.

Although the invention has been described with reference to specific examples, it will be appreciated by those skilled in the art that the invention may be embodied in many other forms.

### Examples

In the following described experimentation, the X-rays used are derived from synchrotron radiation or other monochromatic X-ray sources providing X-rays within the energy range of five to twenty-five keV.

Where the keratin-containing component studied was hair, a single hair was used. The hair was mounted on a holder under sufficient tension to maintain alignment. The holder was mounted on a motorised translation device capable of moving in 1µm steps in the vertical and horizontal planes which enabled each sample to be precisely located in the X-ray beam. The hair fibres were mounted with the axis of the hair in the parallel plane and at a 90 degree angle of incidence to the X-ray source. The wavelength of the X-ray beam was approximately 1.1Å and had a resolution (ΔE/E) of 1x10-4.

Each fibre was exposed to the X-ray source for a set period of time depending on the intensity of the beam. In general each sample was exposed to a total of approximately 1x10¹⁴ photons. The resultant diffraction patterns were collected on a MAR165 CCD detector. Sample to detector distance was approximately 600 mm.

### Experiments

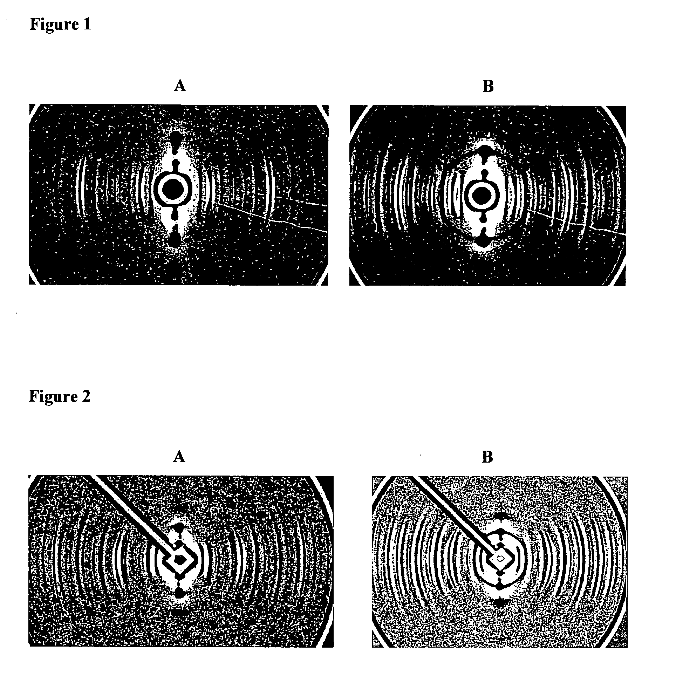

To determine that the ring found in a sample of hair from a breast cancer patient (as shown in Fig 1B) is lipid-derived and, if so, to characterise the lipid.

From the applicant's previous studies, it is evident that certain conditions such as breast cancer, colon cancer and Alzheimer's disease result in the alteration of a keratin-containing component such as hair. This alteration may be detected using the X-ray diffraction technique outlined above. In patients suffering from breast cancer, a ring is observed. The diffraction pattern from a sample of hair from a healthy person is shown in Fig. 1A and from a breast cancer patient is shown in Figure 1B. The ring reported to be associated with the presence of breast cancer can be seen in the diffraction pattern in Fig 1B. To date, it has been unclear what causes this ring to appear. It has been speculated that it is due to an 'additional component' secreted from the breast cancer and taken up by the hair. The present invention addresses this by the finding that the ring is due to the presence of additional lipids or an increase in the amount of a particular lipid incorporated into the structure of keratin-containing components (see below).

### Experiment 1

### Addition of fatty acids to hair

A sample of hair which did not have a ring (confirmed by X-ray diffraction) was selected. The diffraction pattern of this hair is shown in Figure 2A.

The hair was then soaked in olive oil for 10 minutes, wiped dry and then re-exposed to X-rays. The resulting diffraction pattern is depicted in Figure 2B wherein a clear ring is visible. The ring resulting from soaking in olive oil is very similar in appearance and d-spacing to the ring shown in Figure 1B from a breast cancer patient.

### Experiment 2

### Solvent extraction of hairs

Hairs from breast cancer patients which displayed rings were run on a synchrotron SAXS beamline (Australian Synchrotron) to confirm the presence of the ring. The individual hairs were then soaked in a solvent for 2 hours, rinsed in MilliQ water, dried and then re-run very close to the same point on the hair fibre. The solvents used were:
A Isopropanol (100%);
B Chloroform (100%)
C Chloroform/methanol (1:1);
D Chloroform/methanol (1:2)

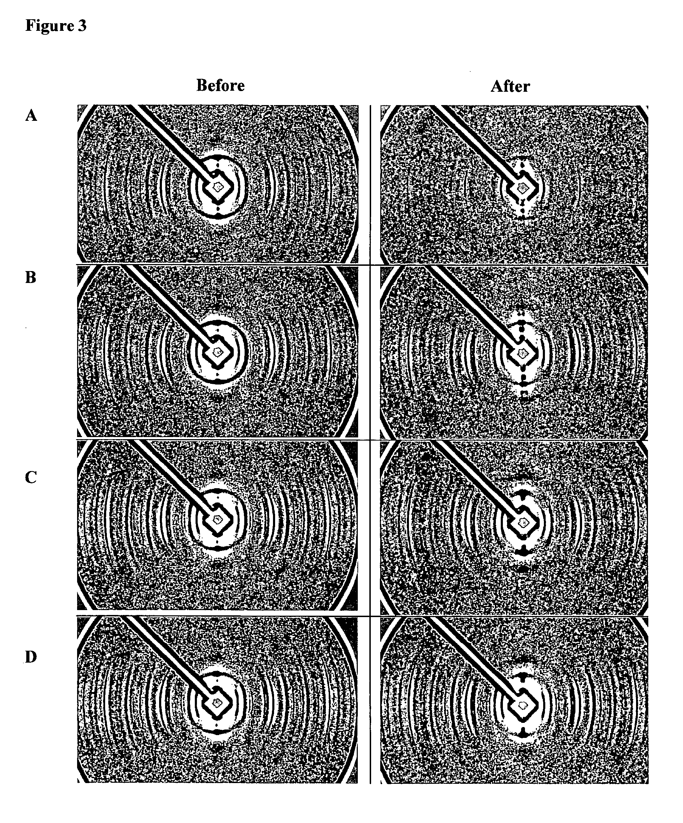

The results are shown in Figure 3.

While all solvents reduced the intensity of the ring in most fibres, it was evident that consistently, isopropanol was the least effective and chloroform/methanol (1:2) was the most effective. This provided data to point to the species of lipid involved.

Particularly, chloroform/methanol in this ratio is used to extract neutral lipids, diacylglcerophospholipids and most sphingolipids from biological material with low lipid content.

### Experiment 3

### Enhancement of the Lipid Ring using Lead Salts

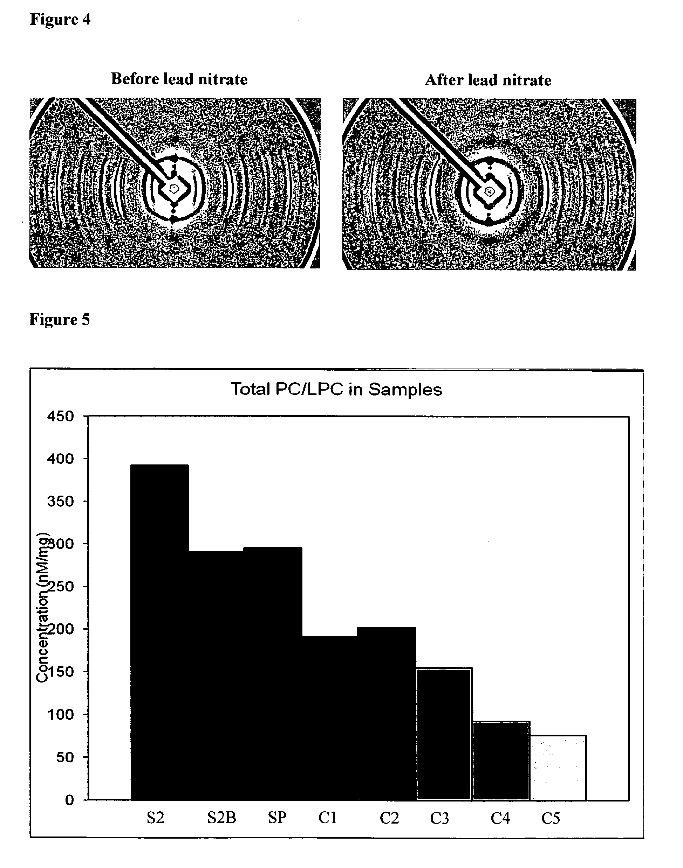

Hairs were soaked in 0.1 mol/l lead nitrate, at pH 5.8, at room temperature for 2 hours. Hairs were thoroughly washed in three successive 5 min immersions in MilliQ water, using the method of Bertrand et al (Bertrand L, Doucet J, Simionovici A, Tsoucaris G, Walter P, 2003. Lead revealed lipid organization in human hair BBA 1620: 218-224.). The results showed that the breast cancer ring was significantly enhanced in most cases. Figure 4 shows the diffraction pattern of a sample hair before and after lipid enhancement by lead nitrate. The ring after enhancement is much stronger than that before exposure to the lead nitrate.

### Experiment 4

### Characterisation of lipid extracted from hair

Samples used in this study were:
- S2: pooled Patient Nos 11018 and 11019 - stage 2 breast cancer (split into replicates S2-1 and S2-2)
- S2B: pooled Patient Nos 11023 and 11010 - stage 2B breast cancer
- SP: pooled Patient Nos 11009, 11034 and 11029 - stages 4, 2A and unknown respectively
- Healthy controls C1 to C5 (C1 split into replicates C1-1 and C1-2, C2 split into replicates C2-1 to C2-3, and C3 split into replicates C3-1 to C3-2).

Hair samples were washed in acetone for 5-10 minutes. Dried samples were weighed into cryo-mill tubes and 1000µL of extraction solution (methanol, chloroform, water (2:1:0.6)) was added into each tube. Samples were spiked with 10µl of 100µM internal standard (Cholesterol ester (18:0)d) to 10µM final concentration. Hair was ground using a cryo-mill (Precellys) with 1.4mm ceramic beads at 6800rpm for three times of 30 seconds with 45 second between intervals and the samples were centrifuged for 10 minutes at 10000rpm. Supernatant (500µL) was transferred into eppendorf tubes and dried in speed-vac. Samples were resuspended in 100µl of n-butanol and methanol (v/v 1:1), centrifuged for 10 minutes at 10000rpm and 50µL of supernatant transferred to high-performance liquid chromatography (HPLC) vials for liquid chromatography mass spectrometry (LCMS) analysis.

Lipids were separated by injecting 5µL aliquots onto a 50mm × 2.1mm × 2.7µm Ascentis Express RP Amide column (Supelco) using an Agilent LC 1200. Lipid samples were eluted at 0.2 mLmin⁻¹ over a 5 min gradient of water/methanol/tetrahydrofuran (50:20:30, v/v/v) to water/methanol/tetrahydrofuran (5:20:75, v/v/v), with the final buffer held for 3 min. Lipids were analysed by electrospray ionisation-mass spectrometry (ESI-MS) using an Agilent Triple Quad 6460. The mass spectrometer in the positive mode was set to scan in precursor ion mode to identify phosphatidylcholines (precursors of m/z 184.1), sphingomyelins (precursors of m/z 184.1), ceramides (precursors of m/z 264.6), cholesterol esters precursors of (m/z 369.4), phosphatidylglycerols (precursors of m/z 189) and in the negative mode phosphatidylinositols (precursors of m/z 241). The mass spectrometer was set to scan in neutral loss mode to identify phosphatidylethanolamines (loss of m/z 141 in the positive ion mode) and phosphatidylserines (loss of m/z 87 in the negative ion mode). Multiple reaction monitoring (MRM) scans were used to quantify the identified lipids with the capillary voltage, fragmentor voltage, and collision energy were 4000 V, 140 - 380 V, and 15-60 V, respectively. In all cases, the collision gas was nitrogen at 7 Lmin⁻¹.

LCMS data was processed using Agilent MassHunter quantitative software and determined the mass and levels of the lipids extracted from the hair of breast cancer patients and healthy controls.

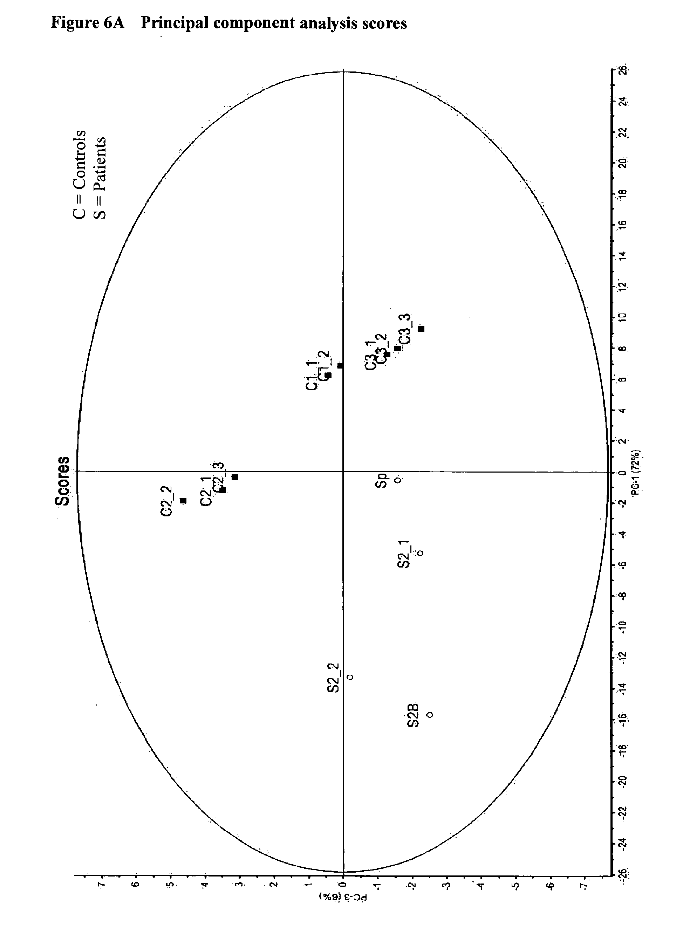

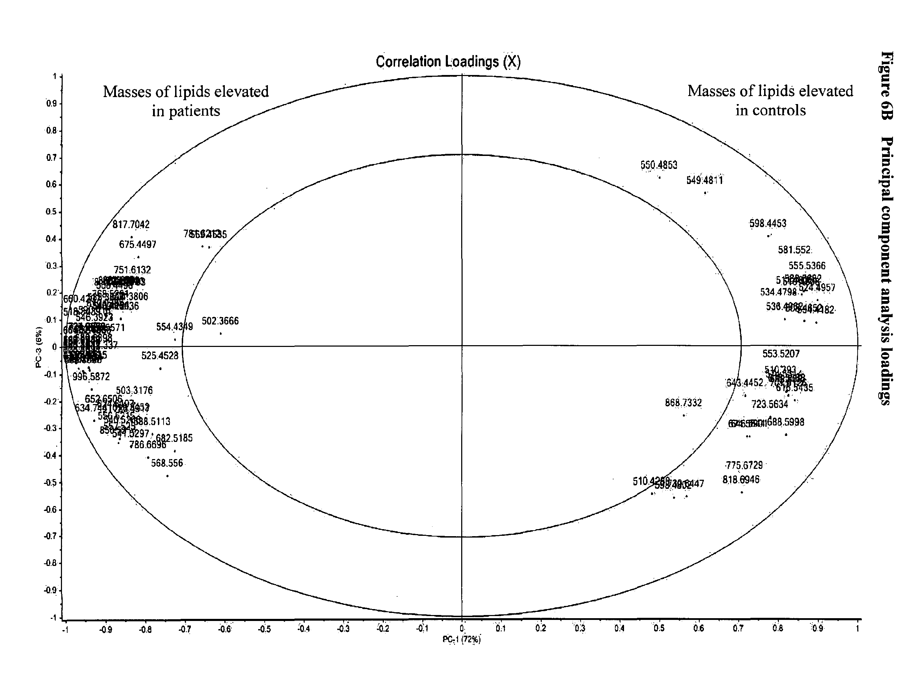

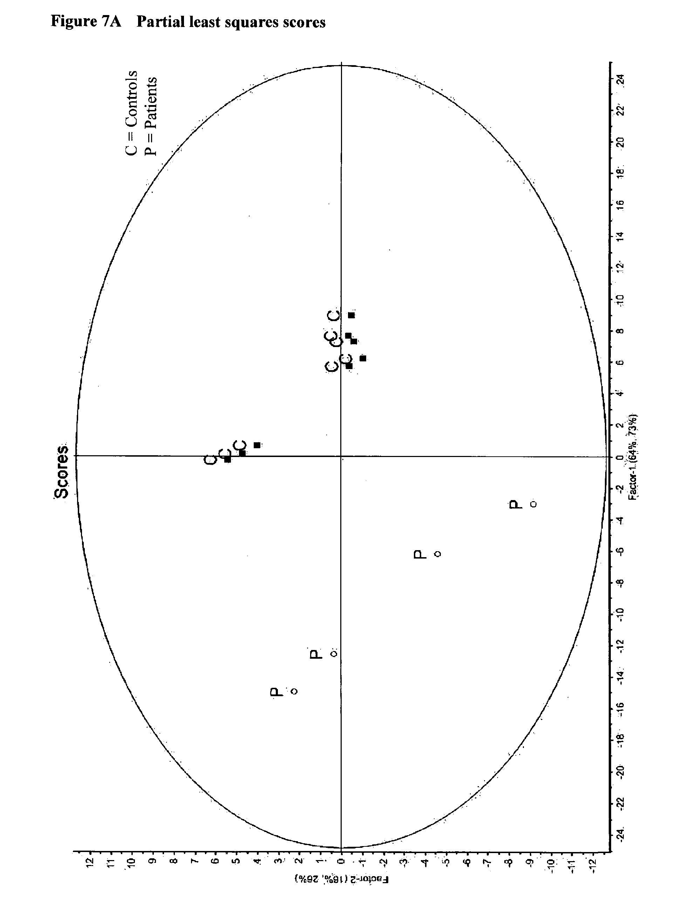

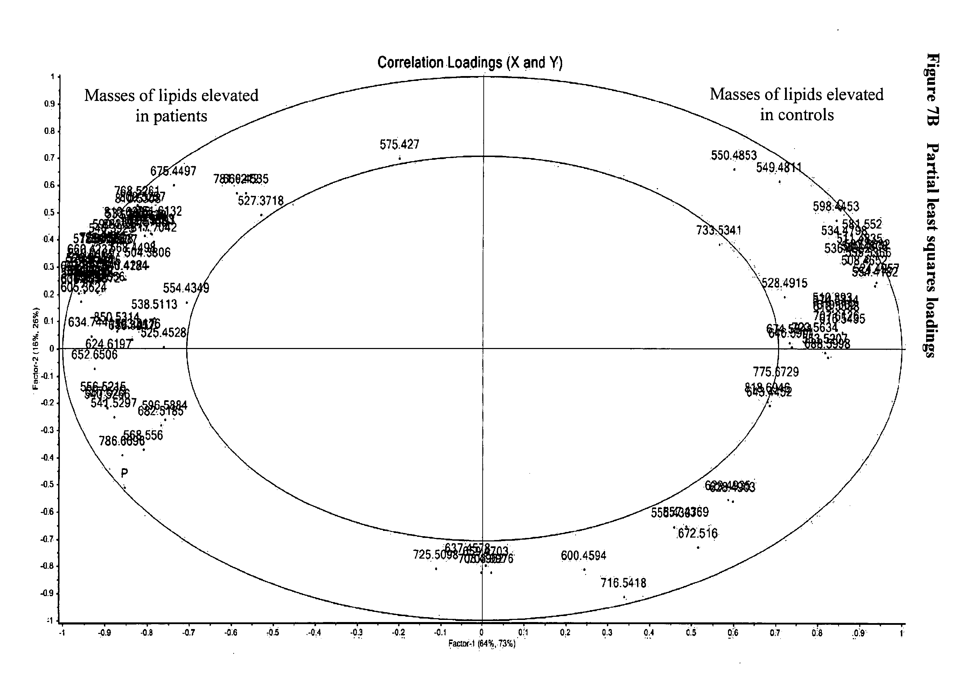

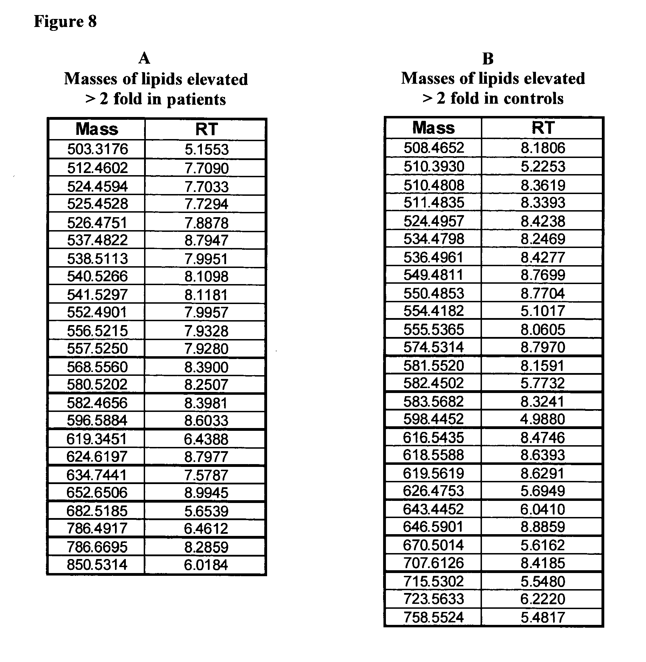

The results showed that the hair of breast cancer patients has higher levels of certain lipids (phosphatidylcholine/lysophosphatidylcholine) than the hair of healthy controls (Figure 5). The principal component analysis scores plot (Figure 6A) and partial least squares analysis scores plot (Figure 7A) showed differences between the lipids extracted from the hair of breast cancer patients and the hair of healthy controls. The principal component analysis loadings plot (Figure 6B) and partial least squares analysis loadings plot (Figure 7B) shows the masses of lipids elevated in breast cancer patients or healthy controls. The masses and retention times of lipids with a >2 fold increase in breast cancer patients or healthy controls are presented in Figure 8.

### Summary

The use of X-ray diffraction to establish the presence of an abnormality, in the form of a ring or halo, suggests that a patient from whom the sample is taken suffers from a condition which affects a keratin-containing component such as a hair. The present inventors have found that the associated ring in the pattern is due to a change in the lipid content of the keratin-containing component.

With this understanding, the inventors have found a method of testing hair for the presence of a component, being one or more lipids which is associated with a condition to thereby provide a reliable diagnostic tool in the early detection of conditions including breast cancer.

Characterisation of the identity and levels of lipids in keratin-containing components of subjects having a condition which changes the lipid profile in the keratin-containing component will enable the identification of lipid profiles that are indicative of diseases such as breast cancer.

## Claims

1. A method of identifying a neoplastic condition in a subject, the method including the steps of:
applying one or more extraction agents to a keratin-containing component from the subject to extract one or more lipids; and
identifying the one or more lipids,
wherein the identity of the one or more lipids is indicative of a subject having the neoplastic condition.

2. A method of identifying a neoplastic condition in a subject, the method including the steps of:
applying one or more extraction agents to a keratin-containing component from the subject to extract one or more lipids; and
analysing the level of the one or more lipids;
wherein the level of the one or more lipids compared to a predetermined standard is indicative of a subject having the neoplastic condition.

3. A method of identifying a neoplastic condition in a subject, the method including the steps of:
applying one or more extraction agents to a keratin-containing component from the subject to extract one or more lipids;
identifying the one or more lipids; and
analysing the level of the one or more lipids;
wherein the identity and level of the one or more lipids compared to a predetermined standard is indicative of a subject having the neoplastic condition.

4. The method according to any one of claims 1 to 3 wherein the neoplastic condition is cancer.

5. The method according to claim 4 wherein the cancer is selected from cancer of the breast, colon, lung, cervix, pancreas, stomach, vagina, oesophagus, kidney, ovary, duodenum, small intestine, rectum, salivary gland, and cecum.

6. The method according to any one of claims 1 to 5 wherein the keratin-containing component is hair.

7. The method according to any one of claims 1 to 6 wherein the extraction agent includes a polar solvent, a non-polar solvent, or a combination thereof.

8. The method according to claim 7 wherein the polar solvent is selected from the group consisting of methanol, ethanol, acetone, n-propanol, isopropanol, butanol, acetic acid, formic acid, dimethyl sulfoxide, dimethylformamide, acetonitrile, tetrahydrofuran and 1,4-dioxane.

9. The method according to claim 7 wherein the non-polar solvent is selected from the group consisting of chloroform, hexane, toluene, benzene, diethyl ether and ethyl acetate.

10. The method according to claim 7 wherein the extraction agent includes chloroform and methanol.

11. The method according to any one of claims 1 or 3 to 10 wherein the identifying the one or more lipids is by thin layer chromatography, mass spectrometry, gas chromatography or high-performance liquid chromatography.

12. The method according to claim 11 wherein the one or more lipids is a phospholipid.

13. The method according to claim 12 wherein the phospholipid is selected from the group consisting of phosphatidylcholine, lysophosphatidylcholine, phosphocholine and phosphatidylethanolamine.

14. The method according to any one of claims 2 to 10 wherein the analysing the level of the one or more lipids compared to a predetermined standard is by thin layer chromatography, mass spectrometry, gas chromatography or high-performance liquid chromatography.

15. The method according to any one of claims 2 to 11 wherein the condition is a condition which results in an increase in the level of the one or more lipids compared to a predetermined standard.

16. The method according to claim 15 wherein the one or more lipids are selected from lipids having mass 503.3176, 512.4602, 524.4594, 525.4528, 526.4751, 537.4822, 538.5113, 540.5266, 541.5297, 552.4901, 556.5215, 557.5250, 568.5560, 580.5202, 582.4656, 596.5884, 619.3451, 624.6197, 634.7441, 652.6506, 682.5185, 786.4917, 786.6695 and 850.5314.

17. The method according to any one of claims 2 to 11 wherein the condition is a condition which results in a decrease in the level of the one or more lipids compared to a predetermined standard.

18. The method according to claim 17 wherein the one or more lipids are selected from lipids having mass 508.4652, 510.3930, 510.4808, 511.4835, 524.4957, 534.4798, 536.4961, 549.4811, 550.4853, 554.4182, 555.5365, 574.5314, 581.5520, 582.4502, 583.5682, 598.4452, 616.5435, 618.5588, 619.5619, 626.4753, 643.4452, 646.5901, 670.5014, 707.6126, 715.5302, 723.5633 and 758.5524.

19. A method of identifying cancer in a subject, the method including the steps of:
applying a mixture of methanol, chloroform and water to hair from the subject to extract one or more lipids;
measuring the mass and level of the one or more lipids by mass spectrometry; and
identifying the one or more lipids,
wherein the identity or level of the one or more lipids is indicative of cancer.

## Patentansprüche

1. Verfahren zur Identifizierung eines neoplastischen Zustands in einem Subjekt, wobei das Verfahren die folgenden Schritte umfasst:
Hinzufügen eines oder mehrerer Extraktionsagenzien zu einem keratinhaltigen Bestandteil von dem Subjekt, um ein oder mehrere Lipide zu extrahieren; und
Identifizieren des einen oder der mehreren Lipide,
wobei die Identität des einen oder der mehreren Lipide auf ein Subjekt mit dem neoplastischen Zustand hinweist.

2. Verfahren zur Identifizierung eines neoplastischen Zustands in einem Subjekt, wobei das Verfahren die folgenden Schritte umfasst:
Hinzufügen eines oder mehrerer Extraktionsagenzien zu einem keratinhaltigen Bestandteil von dem Subjekt, um ein oder mehrere Lipide zu extrahieren; und
Analysieren des Niveaus des einen oder der mehreren Lipide,
wobei das Niveau des einen oder der mehreren Lipide im Vergleich zu einem vorbestimmten Standard auf ein Subjekt mit dem neoplastischen Zustand hinweist.

3. Verfahren zur Identifizierung eines neoplastischen Zustands in einem Subjekt, wobei das Verfahren die folgenden Schritte umfasst:
Hinzufügen eines oder mehrerer Extraktionsagenzien zu einem keratinhaltigen Bestandteil von dem Subjekt, um ein oder mehrere Lipide zu extrahieren;
Identifizieren des einen oder der mehreren Lipide, und
Analysieren des Niveaus des einen oder der mehreren Lipide,
wobei die Identität und das Level des einen oder der mehreren Lipide im Vergleich zu einem vorbestimmten Standard auf ein Subjekt mit dem neoplastischen Zustand hinweist.

4. Verfahren gemäß einem der Ansprüche 1 bis 3, wobei der neoplastische Zustand Krebs ist.

5. Verfahren gemäß Anspruch 4, wobei der Krebs ausgewählt ist aus Brustkrebs, Kolonkrebs, Lungenkrebs, Gebärmutterhalskrebs, Pankreaskrebs, Magenkrebs, Vaginakrebs, Ösophaguskrebs, Nierenkrebs, Eierstockkrebs, Zwölffingerdarmkrebs, Dünndarmkrebs, Rektumkrebs, Speicheldrüsenkrebs und Zökumkrebs.

6. Verfahren gemäß einem der Ansprüche 1 bis 5, wobei der keratinhaltige Bestandteil Haar ist.

7. Verfahren gemäß einem der Ansprüche 1 bis 6, wobei das Extraktionsagens ein polares Lösungsmittel, ein nichtpolares Lösungsmittel oder eine Kombination hiervon umfasst.

8. Verfahren gemäß Anspruch 7, wobei das polare Lösungsmittel ausgewählt ist aus der Gruppe, bestehend aus Methanol, Ethanol, Aceton, n-Propanol, Isopropanol, Butanol, Essigsäure, Ameisensäure, Dimethylsulfoxid, Dimethylformamid, Acetonitril, Tetrahydrofuran und 1,4-Dioxan.

9. Verfahren gemäß Anspruch 7, wobei das nicht-polare Lösungsmittel ausgewählt ist aus der Gruppe, bestehend aus Chloroform, Hexan, Toluol, Benzol, Diethylether und Ethylacetat.

10. Verfahren gemäß Anspruch 7, wobei das Extraktionsagens Chloroform und Methanol umfasst.

11. Verfahren gemäß einem der Ansprüche 1 oder 3 bis 10, wobei das Identifizieren des einen oder der mehreren Lipide mittels Dünnschichtchromatographie, Massenspektrometrie, Gaschromatographie oder Hochleistungsflüssigkeitschromatografie geschieht.

12. Verfahren gemäß Anspruch 11, wobei das eine oder die mehreren Lipide ein Phospholipid ist.

13. Verfahren gemäß Anspruch 12, wobei das Phospholipid ausgewählt ist aus der Gruppe, bestehend aus Phosphatidylcholin, Lysophosphatidylcholin, Phosphocholin und Phosphatidylethanolamin.

14. Verfahren gemäß einem der Ansprüche 2 bis 10, wobei das Analysieren des Niveaus des einen oder der mehreren Lipide im Vergleich zu einem vorbestimmten Standard mittels Dünnschichtchromatographie, Massenspektrometrie, Gaschromatographie oder Hochleistungsflüssigkeitschromatografie geschieht.

15. Verfahren gemäß einem der Ansprüche 2 bis 11, wobei der Zustand ein Zustand ist, welcher eine Erhöhung des Niveaus des einen oder der mehreren Lipide im Vergleich zu einem vorbestimmten Standard ergibt.

16. Verfahren gemäß Anspruch 15, wobei das eine oder die mehreren Lipide ausgewählt sind aus Lipiden mit einer Masse von 503,3176, 512,4602, 524,4594, 525,4528, 526,4751, 537,4822, 538,5113, 540,5266, 541,5297, 552,4901, 556,5215, 557,5250, 568,5560, 580,5202, 582,4656, 596,5884, 619,3451, 624,6197, 634,7441, 652,6506, 682,5185, 786,4917, 786,6695 und 850,5314.

17. Verfahren gemäß einem der Ansprüche 2 bis 11, wobei der Zustand ein Zustand ist, welcher eine Verringerung des Niveaus des einen oder der mehreren Lipide im Vergleich zu einem vorbestimmten Standard ergibt.

18. Verfahren gemäß Anspruch 17, wobei das eine oder die mehreren Lipide ausgewählt sind aus Lipiden mit einer Masse von 508,4652, 510,3930, 510,4808, 511,4835, 524,4957, 534,4798, 536,4961, 549,4811, 550,4853, 554,4182, 555,5365, 574,5314, 581,5520, 582,4502, 583,5682, 598,4452, 616,5435, 618,5588, 619,5619, 626,4753, 643,4452, 646,5901, 670,5014, 707,6126, 715,5302, 723,5633 und 758,5524.

19. Verfahren zur Identifizierung von Krebs in einem Subjekt, wobei das Verfahren die folgenden Schritte umfasst:
Hinzufügen einer Mischung aus Methanol, Chloroform und Wasser zu Haar des Subjekts, um ein oder mehrere Lipide zu extrahieren;
Messen der Masse und des Niveaus des einen oder der mehreren Lipide mittels Massenspektrometrie; und
Identifizieren des einen oder der mehreren Lipide,
wobei die Identität oder das Niveau des einen oder der mehreren Lipide auf Krebs hinweist.

## Revendications

1. Procédé d'identification d'une affection néoplasique chez un sujet, le procédé comportant les étapes consistant :
à appliquer un ou plusieurs agent(s) d'extraction sur un composant contenant de la kératine à partir du sujet afin d'extraire un ou plusieurs lipide(s) ; et
à identifier le ou les plusieurs lipide(s), où l'identité du ou des plusieurs lipide(s) est indicative d'un sujet ayant une affection néoplasique.

2. Procédé d'identification d'une affection néoplasique chez un sujet, le procédé comportant les étapes consistant :
à appliquer un ou plusieurs agent(s) d'extraction sur un composant contenant de la kératine à partir du sujet afin d'extraire un ou plusieurs lipide(s) ; et
à analyser le niveau du ou des plusieurs lipide(s) ; où le niveau du ou des plusieurs lipide(s) par rapport à une norme prédéterminée est indicatif d'un sujet ayant une affection néoplasique.

3. Procédé d'identification d'une affection néoplasique chez un sujet, le procédé comprenant les étapes consistant :
à appliquer un ou plusieurs agent(s) d'extraction à un composant contenant de la kératine à partir du sujet afin d'extraire un ou plusieurs lipide(s) ;
à identifier un ou plusieurs lipide(s) ; et
à analyser le niveau de l'un ou de les plusieurs lipide(s),
où l'identité et le niveau du ou des plusieurs lipide(s) par rapport à une norme prédéterminée sont indicatifs d'un sujet ayant une affection néoplasique.

4. Procédé selon l'une quelconque des revendications 1 à 3, dans lequel l'affection néoplasique est un cancer.

5. Procédé selon la revendication 4, dans lequel le cancer est choisi parmi le cancer du sein, du côlon, du poumon, du col de l'utérus, du pancréas, de l'estomac, du vagin, de l'oesophage, des reins, des ovaires, du duodénum, de l'intestin grêle, du rectum, des glandes salivaires, et de caecum.

6. Procédé selon l'une quelconque des revendications 1 à 5, dans lequel le composant contenant de la kératine correspond aux cheveux.

7. Procédé selon l'une quelconque des revendications 1 à 6, dans lequel l'agent d'extraction comporte un solvant polaire, un solvant non polaire, ou une combinaison de ceux-ci.

8. Procédé selon la revendication 7, dans lequel le solvant polaire est choisi dans le groupe constitué de méthanol, d'éthanol, d'acétone, de n-propanol, d'isopropanol, de butanol, d'acide acétique, d'acide formique, de diméthylsulfoxyde, de dimethylformamide, d'acétonitrile, de tétrahydrofurane et de 1,4-dioxane.

9. Procédé selon la revendication 7, dans lequel le solvant non polaire est choisi dans le groupe constitué de chloroforme, d'hexane, de toluène, de benzène, d'éther diéthylique et d'acétate d'éthyle.

10. Procédé selon la revendication 7, dans lequel l'agent d'extraction comporte du chloroforme et du méthanol.

11. Procédé selon l'une quelconque des revendications 1 ou 3 à 10, dans lequel l'identification du ou des plusieurs lipide(s) est par chromatographie sur couche mince, spectrométrie de masse, chromatographie en phase gazeuse ou chromatographie en phase liquide à haute performance.

12. Procédé selon la revendication 11, dans lequel le ou les plusieurs lipide(s) est un phospholipide.

13. Procédé selon la revendication 12, dans lequel le phospholipide est choisi dans le groupe constitué de phosphatidylcholine, de lysophosphatidylcholine, de phosphocholine et de phosphatidyléthanolamine.

14. Procédé selon l'une quelconque des revendications 2 à 10, dans lequel l'analyse du niveau du ou des plusieurs lipide(s) par rapport à une norme prédéterminée est par chromatographie sur couche mince, par spectrométrie de masse, par chromatographie en phase gazeuse ou par chromatographie en phase liquide à haute performance.

15. Procédé selon l'une quelconque des revendications 2 à 11, dans lequel l'affection est une affection qui résulte en une augmentation dans le niveau du ou des plusieurs lipide(s) par rapport à une norme prédéterminée.

16. Procédé selon la revendication 15, dans lequel le ou les plusieurs lipide(s) est/sont choisi(s) parmi les lipides ayant une masse de 503,3176, 512,4602, 524,4594, 525,4528, 526,4751, 537,4822, 538,5113, 540,5266, 541,5297, 552,4901, 556,5215, 557,5250, 568,5560, 580,5202, 582,4656, 596,5884, 619,3451, 624,6197, 634,7441, 652,6506, 682,5185, 786,4917 786,6695 et de 850,5314.

17. Procédé selon l'une quelconque des revendications 2 à 11, dans lequel l'affection est une affection qui résulte en une diminution dans le niveau du ou des plusieurs lipide(s) par rapport à une norme prédéterminée.

18. Procédé selon la revendication 17, dans lequel le ou les plusieurs lipide(s) sont choisis parmi les lipides ayant une masse de 508,4652, 510,3930, 510,4808, 511,4835, 524,4957, 534,4798, 536,4961, 549,4811, 550,4853, 554,4182, 555,5365, 574,5314, 581,5520, 582,4502, 583,5682, 598,4452, 616,5435, 618,5588, 619,5619, 626,4753, 643,4452, 646,5901, 670,5014, 707,6126, 715,5302 723,5633 et de 758,5524.

19. Procédé d'identification d'un cancer chez un sujet, le procédé comportant les étapes consistant :
à appliquer un mélange de méthanol, de chloroforme et d'eau sur les cheveux à partir du sujet pour extraire un ou plusieurs lipide(s) ;
à mesurer la masse et le niveau du ou des plusieurs lipide(s) par spectrométrie de masse ; et
à identifier le ou les plusieurs lipide(s), où l'identité ou le niveau du ou des plusieurs lipide(s) est indicatif d'un cancer.
